# EUROPEAN PATENT APPLICATION

(11) **EP 3 521 450 A1**
(43) Date of publication of application: **07.08.2019**
(21) Application number: 17856745.9
(22) Date of filing: 27.09.2017
(51) Int. Cl.: C12Q 1/68, G01N 33/68

(54) **METHOD FOR DETECTING SMALL RNAS OR PROTEINS ASSOCIATED WITH SMALL RNAS**

(30) Priority: 27.09.2016 KR 20160123710
(71) Applicant: Nuribio Co., Ltd., Suwon-si, Gyeonggi-do 16229 (KR)
(72) Inventor: NAM, Young Hyean, Suwon-si Gyeonggi-do 16243 (KR)
(74) Representative: Mewburn Ellis LLP
(86) International application number: PCT/KR2017/010737
(87) International publication number: WO 2018/062859

(57) **Abstract**

The present invention relates to a method for detecting small RNA or small RNA-associated protein, and more particularly to a method of detecting small RNA or small RNA-associated protein using a promer, which has the structure X-Y-Z and comprises a nucleotide sequence capable of binding complementarily to the whole or a part of the small RNA to be detected. The present invention makes it possible to rapidly and accurately detect the small RNA or the small RNA-associated protein, and thus may be advantageously used for diagnosis of various diseases and prognostic prognosis.

<110>     Nuribio Co., Ltd.

<120>     Detecting method for small RNA or protein associated with small
          RNA

<130>     FP-17-0117-PCT

<150>     KR 10-2016-0123710
<151>     2016-09-27

<160>     12

<170>     KoPatentIn 3.0

<210>     1
<211>     25
<212>     DNA_RNA
<213>     Artificial Sequence

<220>
<223>     miRNA-21 promer


<220>
<223>     RNA is located in 23th of sequence.


<400>     1
ccctgtagct tatcagactg atgtt                                          25


<210>     2
<211>     18
<212>     DNA
<213>     Artificial Sequence

<220>
<223>     miRNA-21 reverse primer


<400>     2
gccgctgagg tagtaggt                                                  18


<210>     3
<211>     29
<212>     DNA
<213>     Artificial Sequence

<220>
<223>     miRNA-21 reverse transcription primer


<400>     3
gccgctgagg tagtaggttg tgtttttca                                      29


<210>     4
<211>     26
<212>     DNA_RNA
<213>     Artificial Sequence

<220>

<223>    miRNA-155 promer

<220>
<223>    RNA is located in 24th of sequence.

<400>    4
ggctgttaat gctaatcgtg atcggg                                          26

<210>    5
<211>    18
<212>    DNA
<213>    Artificial Sequence

<220>
<223>    miRNA-155 reverse primer

<400>    5
gccgctgagg tagtaggt                                                   18

<210>    6
<211>    29
<212>    DNA
<213>    Artificial Sequence

<220>
<223>    miRNA-155 reverse transcription primer

<400>    6
gccgctgagg tagtaggttg tgttttacc                                       29

<210>    7
<211>    24
<212>    DNA_RNA
<213>    Artificial Sequence

<220>
<223>    let-7a promer

<220>
<223>    RNA is located in 22th of sequence.

<400>    7
gctgcttgag gtagtaggtt guat                                            24

<210>    8
<211>    18
<212>    DNA
<213>    Artificial Sequence

<220>
<223>    let-7a reverse primer

<400>    8
gccgctgagg tagtaggt                                                    18


<210>    9
<211>    28
<212>    DNA
<213>    Artificial Sequence

<220>
<223>    let-7a reverse transcription primer


<400>    9
gctaacgtct gtacttcgtc atttaact                                         28


<210>    10
<211>    24
<212>    DNA_RNA
<213>    Artificial Sequence

<220>
<223>    let-7d promer


<220>
<223>    RNA is located in 22th of sequence.


<400>    10
gctgctagag gtagtaggtt gcat                                             24


<210>    11
<211>    18
<212>    DNA
<213>    Artificial Sequence

<220>
<223>    let-7d reverse primer


<400>    11
gccgctgagg tagtaggt                                                    18


<210>    12
<211>    28
<212>    DNA
<213>    Artificial Sequence

<220>
<223>    let-7d reverse transcription primer


<400>    12
gctaacgtct gtacttcgtc atttaact                                         28

## Description

### [Technical Field]

The present invention relates to a method for detecting small RNA or small RNA-associated protein, and more particularly to a method of detecting small RNA or small RNA-associated protein using a promer, which has the structure X-Y-Z and comprises a nucleotide sequence capable of binding complementarily to the whole or a part of the small RNA to be detected.

### [Background Art]

Micro RNA (miRNA), one of small RNAs present in cells, is known to play a very important role in biological processes, such as cellular development and differentiation and programmed cell death, and a difference in the expression levels of particular miRNAs in cells is known to be associated with various cancers, including cancer. Therefore, the detection of particular microRNAs (miRNAs) has recently become an important part in the scientific research field. Specifically, detection and identification of particular miRNAs and proteins associated therewith can be used as genetic and biological markers that indicate human health. Through the detection and identification, it has become possible to develop kits that simply use peripheral blood, hormones or spinal fluid, and diagnostic kits which can be used *in vitro.* These kits can be applied to diagnosis of dementia diseases (Alzheimer's disease, Parkinson's disease, etc.), diabetes, cancer, and the like. However, miRNAs are very short in length (about 22 nucleotides), and thus are difficult to detect and identify, limiting their use as effective biomarkers.

Detection of miRNAs is generally based on a nucleic acid amplification method, and examples of this method include PCR (polymerase chain reaction), NASBA (nucleic acid sequence based amplification), and the like. A conventional detection method based on this nucleic acid amplification reaction is known to those skilled in the art to which the present invention pertains. This detection method usually requires a labor-intensive processes, such as the use of an electrophoresis, probe or blotting assay technique which can detect an amplified nucleic acid after nucleic acid amplification.

Real-time methods for miRNA detection, which are most widely known, are performed by RT-PCR, qRCR, or the like. These methods are based on a fluorescent hybridization probe which forms a secondary structure whose fluorescence is quenched when not hybridized to a target, a hydrolysis probe whose fluorescence increases due to cleavage by Taq polymerase after hybridization, dsDNA-binding agents, or the like. At each time point of measurement, each amplification product is unfolded or cut as the probe hybridize thereto, thus increasing the fluorescence intensity. In this case, the amplifier is detected at a rate of one amplifier per probe. In a given cycle, one amplifier shows one probe (e.g., molecular beacon, Taqman probe, MGB probe, etc.) detected by hybridization or cleavage of the probe. In addition, among real-time detection methods, a method of detecting an NASBA product using a molecular beacon simultaneously with amplification is also known.

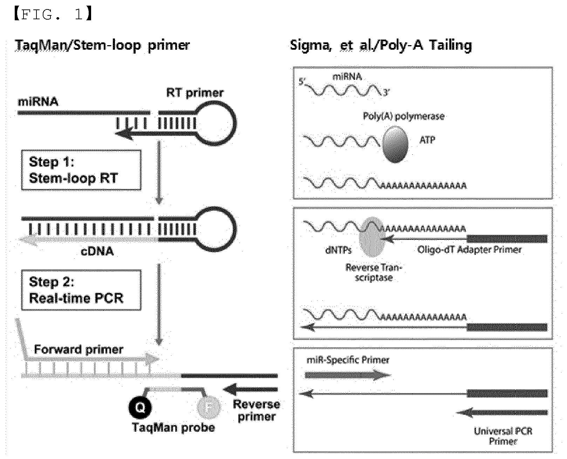

As specific examples of this method for detecting miRNA, as shown in FIG. 1, a method that uses a TaqMan probe and a stem-loop primer and a method that uses an oligo-dT adapter primer and poly-A tailing are widely used.

However, the above-described techniques have some disadvantages.
First, these techniques require additional oligo extension, because a probe and primers for miRNA analysis are not located in the target miRNA. The stem-loop primer, oligo-dT adapter primer, etc., shown in FIG. 1 are extended oligos. In this case, the crucial problem is that the increase in fluorescence that depends entirely on the amplified miRNA target is not measured, but the increase in fluorescence caused by amplification of the extended oligo is measured, which results in inaccurate results due to non-specific amplification and binding.
Second, a certain spacing between the primer and the probe is required. For example, the Taq-man probe and the MGB probe are labeled with a donor chromophore at the 5' end, and when DNA polymerase encounters the probe during primer extension, the exonuclease activity of the polymerase will sequentially degrade the probe starting at the 5' end. At this time, cleavage is possible when the spacing between the primer and the probe is at least 7 to 10 nucleotides. In addition, the spacing between the primer and the quencher at the 3' end requires at least 2 to 4 nucleotides. These limitations play a limited role in the analysis of short RNA or DNA.
Third, the method based on NASBA requires precise control of the melting temperature of the beacon, since the probe must form a secondary structure that quenches the fluorescence intensity of the donor. This control is difficult to apply when a reaction is performed at a constant temperature, like the case of NASBA or RCA. Furthermore, the beacon should be designed such that it maintains the hairpin structure when not binding to the target and is unfolded at a reaction temperature at which it binds to the target. This makes it very difficult to design the probe, and also causes problems associated with signal-to-noise because the probe often emits background fluorescence when the beacon is unfolded at the reaction temperature.
Fourth, it is difficult to distinguish isoforms of small RNAs such as miRNAs. In the case of miRNAs, there are many different isoforms having only 1 to 3 different nucleotides due to their short lengths. For example, similar isoforms of miRNAs, such as let-7, miR-18 and miR-30, are known to be difficult to distinguish.

Accordingly, the present inventors have conducted studies to overcome the above-described disadvantages and to develop a method of rapidly and accurately detecting small RNAs such as miRNA or siRNA, and as a result, have found that small RNA in a very small amount of a sample can be rapidly and accurately detected by hybridizing a promer capable of accurately and rapidly detecting a nucleic acid or a protein, which is a promer described in Korean Patent Application No. 10-2017-0020238 claiming priority based on Korean Patent No. 10-2016-0017359, to small RNA or small RNA cDNA, and then measuring the amount of fluorescent fragments obtained by cleaving the inside of the nucleic acid with a cleavage reagent, thereby completing the present invention.

### [Disclosure]

### [Technical Problem]

It is an object of the present invention to provide a method for detecting small RNA.

Another object of the present invention is to provide a method of detecting small RNA-associated protein using a real-time amplification reaction.

### [Technical Solution]

In one aspect, the present invention provides a promer which is used as a primer and a probe for real-time detection of small RNA or small RNA-associated protein.

The promer is derived from a promer described in Korean Patent Application No. 10-2017-0020238 claiming priority based on Korean Patent Application No. 10-2016-0017359, and the Korean Patent Applications referred to in the present invention are incorporated as part of the specification.

Specifically, in the present invention, the promer may have a structure of X-Y-Z and comprise one or more detectable markers attached to both ends or the inside thereof. Herein, the positions of the attached detectable markers are not limited to particular positions and may be any positions at which the detectable markers are separated when Y of the promer is cleaved by a specific enzyme.

Furthermore, the promer may form a complex by binding to a specific region of the target small RNA or target small RNA-associated protein to be detected in real time. When Y region is cleaved by a specific enzyme, the Y and Z regions of the promer may be separated from the specific region of the target small RNA or target small RNA-associated protein, but the X region is not separated and retains the complex and is used as a primer for amplification.

The nucleic acid structure developed by the present inventors was termed "promer" in the present invention. Hereinafter, unless otherwise specified, the term "promer" refers to the nucleic acid structure that can be used as both a primer and a probe.

The promer of the present invention has the structure of X-Y-Z, and each of X, Y, and Z may have various numbers of nucleotides.

In one example, the X region of the promer is a DNA or RNA consisting of 1-60 nucleotides, preferably 1-30 nucleotides, more preferably 2-30 nucleotides, even more preferably 3-30 nucleotides, still even more preferably 4-30 nucleotides, still even more preferably 5-30 nucleotides, still even more preferably 6-30 nucleotides, still even more preferably 7-30 nucleotides, still even more preferably 8-30 nucleotides, still even more preferably 10-30 nucleotides. If the X region of the promer comprises more than 60 nucleotides, there will be a problem in that a nonspecific reaction occurs during real-time detection of nucleic acid or protein.

In one example, the Y region of the promer is a DNA or RNA consisting of 1-10 nucleotides, preferably 1-9 nucleotides, more preferably 1-8 nucleotides, even more preferably 1-7 nucleotides, still even more preferably 1-6 nucleotides, still even more preferably 1-5 nucleotides, still even more preferably 1-4 nucleotides, still even more preferably 1-3 nucleotides, still even more preferably 1-2 nucleotides. When the Z region of the promer comprises no nucleotide sequence, the Y region of the promer consists of at least 3 nucleotides, preferably 3-10 nucleotides, more preferably 3-9 nucleotides, even more preferably 3-8 nucleotides, still even more preferably 3-7 nucleotides, still even more preferably 3-6 nucleotides, still even more preferably 3-5 nucleotides, still even more preferably 3-4 nucleotides. When the Z region of the promer comprises one nucleotide sequence, the Y region of the promer consists of at least 2 nucleotides, preferably 2-10 nucleotides, more preferably 2-9 nucleotides, even more preferably 2-8 nucleotides, still even more preferably 2-7 nucleotides, still even more preferably 2-6 nucleotides, still even more preferably 2-5 nucleotides, still even more preferably 2-4 nucleotides, still even more preferably 2-3 nucleotides. If the number of nucleotides forming the Y region of the promer is out of the above-described range, there will be a problem in that a nonspecific reaction occurs during real-time detection of nucleic acid or protein to reduce sensitivity.

In one example, the Z region of the promer is a DNA or RNA consisting of 0-10 nucleotides, preferably 1-10 nucleotides, more preferably 2-10 nucleotides. If the number of nucleotides forming the Z region is out of the above-described range, there will be a problem in that the Z region bound to the target nucleic acid or protein is not separated from the target nucleic acid or protein after cleavage of the Y region, and thus the nucleic acid or protein cannot be detected.

At this time, when any one of X, Y and Z of the promer is DNA, one or more of the other two are RNA. Preferably, X, Y and Z are DNA, RNA and DNA, respectively, or RNA, DNA and RNA, respectively.

In one specific example, when the X region is DNA, any one of Y and Z is RNA. For example, Y is RNA, and Z is DNA. Alternatively, Y is DNA, and Z is RNA. Herein, the number of DNA and RNA may be more than 0, and is as defined above for X, Y and Z.

In another specific example, when X is RNA, any one of Y and Z is RNA. For example, Y is RNA, and Z is DNA. Alternatively, Y is DNA, and Z is RNA. Herein, the number of DNA and RNA may be more than 0, and is as defined above for X, Y and Z.

In still another specific example, when Z is null, any one of X and Y is DNA, and the other one is RNA. Herein, the number of DNA and RNA may be more than 1, and is as defined above for X and Y.

Furthermore, X, Y and Z of the promer may be synthesized so as to be wholly or partially methylated to prevent nonspecific cleavage.

In the present invention, the term "nucleic acid" refers to DNA or RNA to be detected in real time in a sample.

In the present invention, the detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

When a fluorescent pair is used as the detectable marker in the present invention, the fluorescent label and the quencher may be located in the X or Z region or located in the Y region, and the location thereof is not limited to any location. In one example, the fluorescent label may be located in the X region, and the quencher may be located in the Y or Z region.

The promer of the present invention may be used in detection of small RNA or small RNA-associated protein. Specifically, it may be used as: i) an RT primer for synthesizing cDNA from small RNA; ii) a forward primer for amplifying cDNA synthesized from small RNA; iii) a reverse primer for amplifying cDNA synthesized from small RNA; iv) a forward primer and a reverse primer for amplifying cDNA synthesized from small RNA; or v) a probe for real-time detection of a small RNA to be detected or a protein associated therewith.

In this set of embodiments, the promer of the present invention can more accurately detect small RNA or small RNA-associated protein than a probe which is degraded by conventional DNA polymerase, because region Y of the promer is cleaved by an enzyme that cleaves region Y of the promer, and then regions Y and Z are separated, and region X can maintain a complex with a target small RNA or a protein associated therewith and can be used as a primer for synthesizing and amplifying the small RNA, and only region Y of the promer is cleaved by the enzyme that cleaves region Y of the promer.

In particular, when a fluorophore or a quencher is bound to the 3' end of region Y or Z of the promer of the present invention, errors caused by nonspecific amplification can be reduced, because the target nucleic acid or the specific nucleic acid of the target protein can be prevented from being amplified by polymerase, as long as region Y of the promer is not cleaved.

Accordingly, when the promer of the present invention is used to detect small RNA or small RNA-associated protein, the analysis time and cost can be reduced, and the small RNA or small RNA-associated protein to be detected can be more accurately and specifically detected compared to a conventional method. Thus, the promer of the present invention may be used as a kit for real-time detection of small RNA or small RNA-associated protein.

Where the promer of the present invention is used as a kit for real-time detection of small RNA or small RNA-associated protein, the kit preferably further comprises, in addition to the promer of the present invention, an enzyme capable of cleaving the Y region of the promer.

In the present invention, the enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABC nuclease. When the Y region is RNA, the enzyme capable of cleaving the Y region is preferably ribonuclease (RNase), specifically RNase II, RNase III, RNase IV, RNase H, or RNase T₂.

Where the promer of the present invention is used as a kit for real-time detection of small RNA or small RNA-associated protein, the kit may further comprise, in addition to the promer of the present invention and the enzyme capable of cleaving the Y region of the promer, reagents required for amplification of DNA.

The reagents required for amplification include, for example, suitable amounts of DNA polymerase (e.g., thermostable DNA polymerase derived from *Thermus aquatiucs* (Taq), *Thermus thermophilus* (Tth), *Thermus filiformis, Thermis flavus, Thermococcus literalis* or *Phyrococcus furiosis* (Pfu)), DNA polymerase cofactor (Mg²⁺), buffer, dNTPs (dATP, dCTP, dGTP and dTTP) and water (dH₂O). In addition, the buffer includes, but is not limited to, suitable amounts of Triton X-100, dimethylsufoxide (DMSO), Tween 20, nonidet P40, PEG 6000, formamide and bovine serum albumin (BSA).

In another aspect, the present invention provides a method for detecting small RNA.

Hereinafter, each step of the method for detecting small RNA according to the present invention will be described in detail.

Step (a) is a step of obtaining the template nucleic acid from biological sample.

In the present invention, the template nucleic acid refers an RNA including a small RNA to be detected in a biological sample. The small RNA is expressed in order to produce a protein or control protein production in a situation where the life phenomenon of the living body needs to be maintained or controlled.

The biological sample may be selected from among various types of samples obtained from an individual, specifically, solid tissue samples, liquid tissue samples, biological liquids, bronchial aspirates, cells, and cell fragments. Specific examples of the biological sample include, but are not limited to, solid tissue samples removed from an individual during surgery, pathological samples, preserved samples, biopsy samples, tissue cultures or cells derived therefrom and fragments prepared from the progeny thereof.

The stored biological sample may be one stored by any conventional method known in the art. The sample may be one stored for more than 1 week or more than 1 year, for example, 1 to 10 years. Alternatively, the sample may be one derived from freeze-stored tissue or from formalin-fixed tissue stored at room temperature.

In the present invention, extraction of RNA from the sample may be performed using various methods known in the art. In one example, it may be performed using trizol or Triton X-100.

Step (b) is a step of synthesizing cDNA from small RNA.

Specifically, step (b) in the method of the present invention is a step of adding an RT primer to the small RNA, extracted in step (a), to synthesize cDNA.

In the present invention, the RT primer is a nucleic acid, which can complementarily bind to a portion of the nucleotide sequence of the RNA to be detected. Herein, the RT primer may be either an RT primer generally known in the art, or the promer of the present invention.

Step (c) is a step of amplifying cDNA.

Specifically, step (c) of the method of the present invention is a step of adding the promer or a kit comprising the promer of the present invention, and/or a forward primer or reverse primer having a nucleotide sequence complementary to the cDNA, to the cDNA synthesized in step (b), and amplifying the cDNA by extension.

The kit comprising the promer refers to a kit comprising the promer of the present invention and an enzyme capable of cleaving the Y region of the promer.

The enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABSC nuclease. When the Y region is RNA, the enzyme capable of cleaving the Y region is preferably ribonuclease (RNase), specifically RNase II, RNase III, RNase IV, RNase H, or RNase T₂.

In the present invention, the promer comprises a nucleotide sequence that can complementarily bind to a portion of the nucleotide sequence of the cDNA synthesized in step (b). To amplify the cDNA, the promer may be used as: i) a forward primer and a probe; or ii) a reverse primer or a probe; or iii) a forward primer, a reverse primer and a probe. Furthermore, the promer of the present invention has a structure of X-Y-Z and comprises one or more detectable markers attached to both ends or the inside thereof. When the Y region of the promer is cleaved by a specific enzyme, the Y and Z regions are separated from the template, and the X region serves as a primer without separation from the template. The structure of such promer is as described above.

The above detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

In one specific example, where the promer of the present invention is to be used as a forward primer and a probe or as a reverse primer and a probe, the promer may comprise a nucleotide sequence capable of complementarily binding to a portion of the nucleotide sequence of the cDNA synthesized in step (b).

In another specific example, where the promer of the present invention is used as a forward primer and a probe or as a reverse primer or a probe, a separate reverse primer or forward primer may be used in addition to the promer. Herein, the separate reverse primer or forward primer is a DNA that can complementarily bind to the cDNA synthesized in step (b) and that comprises 5-30 nucleotides, preferably 10-30 nucleotides. Herein, the reverse primer may be the same as the RT primer used in step (b).

In still another specific example, where the promer of the present invention is used as a forward primer, a reverse primer and a probe, the promer that is used as the reverse primer may be the same as the promer used in step (b), and the detectable markers attached to both ends or the inside of the promer may be the same as or different from those of the promer used in step b).

In the present invention, amplification of the cDNA is performed at an isothermal temperature at which the cDNA is annealed with (A) the promer of the present invention, which is used as a forward primer and a probe or as a reverse primer and a probe, and a forward primer or reverse primer, which is generally used in the art, or (B) the promer of the present invention, which is used as a forward primer, a reverse primer and a probe, and at which the activity of the enzyme used is not substantially inhibited. As used herein, the term "isothermal temperature" means that there is no thermal cycling, and the term does not necessarily mean physically equivalent temperature.

In one specific example, the isothermal temperature at which amplification in the present invention is performed may be 40°C to 80°C, preferably 55°C to 75°C, more preferably 60°C to 75°C.

In the present invention, amplification of the cDNA may be performed by one method selected from the group consisting of polymerase chain reaction, rolling circle amplification, strand displacement amplification, and nucleic acid sequence-based amplification, but is not necessarily limited thereto.

In the present invention, amplification of the cDNA may be performed using reagents required for amplification, in addition to a kit comprising the promer of the present invention. The reagents required for amplification may include, for example, suitable amounts of DNA polymerase (e.g., thermostable DNA polymerase derived from *Thermus aquatiucs* (Taq), *Thermus thermophilus* (Tth), *Thermus filiformis, Thermis flavus, Thermococcus literalis* or *Phyrococcus furiosis* (Pfu)), DNA polymerase cofactor (Mg²⁺), buffer, dNTPs (dATP, dCTP, dGTP and dTTP) and water (dH₂O). In addition, the buffer may include, but are not limited to, suitable amounts of Triton X-100, dimethylsufoxide (DMSO), Tween 20, nonidet P40, PEG 6000, formamide and bovine serum albumin (BSA).

Step (d) is a step of measuring the amount of fragments of the primer cleaved by the enzyme capable of cleaving the Y region of the promer.

In the present invention, measurement of the amount of fragments of the promer may be performed using various detection methods. Specifically, the amount of fragments of the promer cleaved according to the present invention is preferably measured after completion of RT-PCR or amplification, and may be determined by measuring a change in fluorescence intensity or measuring chemiluminescence.

Measurement of the change in fluorescence intensity or chemiluminescence may be performed using any measurement system capable of detecting a fluorescent label, known in the art. For example, the measurement may be performed using a real-time PCR machine, a TRIAD multimode detector, a Wallac/Victor fluorescence plate reader, a Perkin-Elmer LB50B luminescence spectrometer, LightCycler 96, Applied Biosystems 7500, or Biorad CFX96 real-time PCR thermocycler, but is not limited thereto.

The method for measurement and detection of the amount of fragments of the promer cleaved according to the present invention may vary depending on the kind of label or detectable marker introduced into the promer or a reaction solution.

For example, where the promer, in which a fluorescent label is attached to the end of X region and a quencher is attached to the end of Z region, hybridized with the target small RNA or cDNA, the fluorescence of the fluorescent label attached to the end of X region is greatly reduced by the quencher attached to the end of Z region before the Y and Z regions are cleaved by an enzyme capable of cleaving the Y region. However, when the X region is not separated by the enzyme capable of cleaving the Y region, and only the Y and Z regions are separated from the target small RNA or cDNA and dispersed in the reaction solution, the fluorescence of the fluorescent label attached to the end of X region is increased without being affected by the quencher attached to the end of Z region. At this time, it is possible to detect the target small RNA or cDNA in real time by measuring the increased fluorescence emission of the fluorescent label attached to the end of X region using the above-described apparatus.

In one specific example, cDNA was synthesized using an RT primer that complementarily binds to small RNA, and then the cDNA was amplified using the promer of the present invention, which complementarily binds to the cDNA and has FAM attached thereto, as a forward primer or a reverse primer. As a result, it was shown that an amplification curve appeared during amplification of the cDNA, unlike the case of a cDNA amplified using a conventional forward primer to which a detectable marker was not attached.

The method for detecting small RNA according to the present invention makes it possible to accurately amplify a small RNA, because it uses the nucleic acid having the structure X-Y-Z as both a probe and a primer, unlike a conventional method that uses intercalator-type dsDNA-binding agents, such as a Taqman probe, an MGB probe or Sybr Green. In addition, it has an advantage in that it can accurately detect small RNA in a very small amount of a sample, compared to a conventional art in which the amount of probe degraded by DNA polymerase is measured while the template nucleic acid is amplified.

In one specific example, the method for detecting small RNA according to the present invention was used to analyze human miRNA, and as a result, accurate analysis was possible even when the concentration of the miRNA was diluted 1/10000-fold (see Examples 1 and 2).

In another specific example, the method for detecting small RNA according to the present invention was used to real-time analyze let-7 miRNA, and as a result, let-7 miRNA could be analyzed in a stable, rapid and accurate manner even when let-7 miRNA was present in very small amounts, for example, at a concentration of 1 aM (see Examples 3 and 4).

In still another aspect, the present invention provides a method of detecting a small RNA-associated protein molecule using the nucleic acid.

Specifically, the present invention provides a method for detecting a small RNA-associated protein molecule, comprising the steps of: a) producing an antibody having attached thereto a nucleic acid having a nucleotide sequence complementary to a promer having the structure X-Y-Z; b) combining the antibody produced in step a) with a small RNA-associated protein obtained from a biological sample, thereby forming a protein-antibody complex; c) hybridizing the promer having the structure X-Y-Z to the complex, thereby forming a protein-antibody-promer complex; and d) treating the protein-antibody-promer complex with a cleavage reagent, thereby separating a fragment of the promer from the antibody, and then measuring the amount of the separated promer fragment, thereby detecting the small RNA-associated protein.

Hereinafter, each step of the method for detecting a small RNA-associated protein molecule according to the present invention will be described in detail.

Step a) is a step of producing an antibody having attached thereto a nucleic acid having a nucleotide sequence complementary to a promer having the structure X-Y-Z.

In the present invention, the structure, features and the like of the promer having the structure X-Y-Z are as described above, and thus the detailed description thereof will be omitted below.

In the present invention, the nucleic acid having the sequence complementary to the promer having the structure X-Y-Z is synthesized using the nucleotide sequence of the promer having the structure X-Y-Z through an amplification reaction which is generally used in the art.

In the present invention, the antibody having attached thereto the nucleic acid having the nucleotide sequence complementary to the promer having the structure X-Y-Z has a structure in which the nucleic acid having the nucleotide sequence complementary to the promer having the structure X-Y-Z is connected to the Fc region of the antibody via a linker. Herein, the linker is generally a DNA consisting of 1-10 nucleotides.

To connect the nucleic acid having the nucleotide sequence complementary to the promer having the structure X-Y-Z to the antibody, two methods may generally be used. In the first method, a DNA having a thiol-modified 5' end is linked to the free amino groups of the antibody by use of one reagent selected from the group consisting of succinimidyl-4-(N-maleimidomethl)cyclohexane-1-carboxylate (SMMCC), Sulfo-succinimidyl-4-(N-maleimidomethl)cyclohexane-1-carboxylate (Sulfo-SMCC), n-succinimidyl-3-(2-pyridylthio)propionate (SPDP), N-succinimidyl-6-(3'-(2-pyridyldithio)-propionamido)hexanoate (NHS-Ic-SPDP), and sulfo-succinimidyl-6-(3'-(2-pyridyldithio)-propionaamido)hexanoate (Sulfo-NHS-Ic-SPDP). Such reagents have different spacer lengths and water solubilities. If necessary, for an additional operation, the linking region can be cleaved using a thiolation reagent that releases DNA.

In the second method, a linking region is provided between the antibody and DNA by the tetrameric protein strepavidin, wherein the protein sufficiently forms an irreversible bond with biotin. The free amino groups of the antibody are labeled with biotin by reaction with biotin-N-hydroxysuccinimide. Biotinylation of the DNA may be performed by the use of 5' -biotin phoshporamidite or the reaction of biotin-n-hydroxysuccinimide following an amino group at the 5'-end. A conjugate of DNA, strepavidin and the antibody can be prepared by adding 1 molar equivalent of a DNA-strepavidin conjugate.

According to the above-described method, the antibody having attached thereto the nucleic acid may be allowed to react at 4°C for 1 hour, followed by purification with a Superdex 200 gel column, thereby obtaining an antibody-nucleic acid conjugate.

The antibody prepared according to the present invention can specifically recognize the protein to be detected, while the promer of the present invention can hybridize to the antibody. Thus, the antibody can be easily used for real-time detection of the protein to be detected.

Sep b) is a step of attaching the antibody produced in step a) to a small RNA-associated protein molecule obtained from a biological sample, thereby forming a protein-antibody complex.

In the present invention, the small RNA-associated protein may be selected from the group consisting of antibodies, ligands, natural compounds, extracts, synthetic peptides, and new drug candidates, but is not limited thereto.

In the present invention, the combination of the small RNA-associated protein with the antibody may be achieved by mixing the target protein with the antibody produced in step a).

Step c) is a step of hybridizing the promer having the structure X-Y-Z to the protein-antibody complex, thereby forming a protein-antibody-promer complex.

In the present invention, the hybridization of the promer to the protein-antibody complex may be achieved b by adding the promer to the protein-antibody complex.

Step d) is a step of treating the protein-antibody-promer complex with a cleavage reagent, thereby separating a fragment of the promer from the antibody, and then measuring the amount of the separated promer fragment, thereby detecting the small RNA-associated protein.

In the present invention, the cleavage reagent used may be any reagent which is capable of specifically cleaving only region Y of the promer by an enzyme. For example, the cleavage reagent used may be one selected from the group consisting of deoxyribonuclease (DNase), ribonuclease (RNase), helicase, exonuclease, and endonuclease. Preferably, it may be ribonuclease (RNase).

In the present invention, measurement of the amount of the promer may be performed using various detection methods. Specifically, the amount of the promer fragment separated according to the method of the present invention is preferably measured after completion of real-time amplification or reaction, and may be determined by measuring either a change in fluorescence intensity or chemiluminescence.

The measurement of a change in fluorescence intensity or the measurement of chemiluminescence may be performed using any measurement device known in the art, which can detect a fluorescent label. For example, it may be performed using a TRIAD multimode detector, a Wallac/Victor fluorescence reader, a Perkin-Elmer LB50B luminescence spectrometer, or the like, but is not limited thereto.

The measurement of the amount of the separated promer and the detection method as described above may greatly vary depending on the type of label or detection marker introduced into the promer or the reaction solution.

The method for detecting the target protein according to the present invention has an advantage in that the detection rate and accuracy of the small RNA-associated protein to be detected can be increased by using the promer having the structure X-Y-Z and the antibody having the sequence complementary to the promer having the structure X-Y-Z.

### [Advantageous Effects]

To analyze small RNA according to a conventional art, detection is performed depending on an optionally extended oligo by synthesizing cDNA using the extended oligo. However, the promer having the structure X-Y-Z according to the present invention makes it possible to accurately amplify and detect small RNA without depending on an optionally extended oligo sequence. This can prevent erroneous amplification and detection from being caused by the method that performs detection depends on the optionally extended sequence. In addition, the promer is cleaved only by a cleavage reagent, and thus has an advantage in that it can more accurately measure the amount of the promer to be detected, compared to a conventional probe which is degraded by DNA polymerase.

Therefore, the method of detecting small RNA or small RNA-associated protein using the promer having the structure X-Y-Z according to the present invention can rapidly and accurately detect the small RNA or the small RNA-associated protein, and may be advantageously used for diagnosis of various diseases and prognostic prognosis.

### [Description of Drawings]

FIG. 1 is a schematic view showing a method that uses a TaqMan probe and a stem-loop primer and a method that uses an oligo-dT adapter primer and poly-A tailing, among conventional methods that are used to detect small RNA.

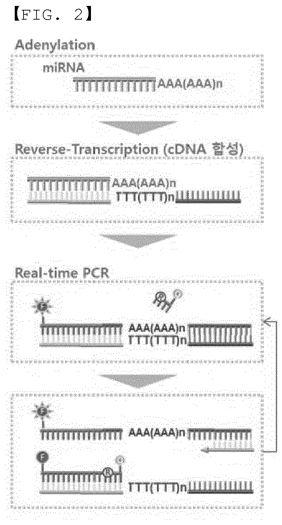

FIG. 2 is a schematic view showing a method in which cDNA is synthesized using an elongated oligo dT primer after poly-(A) tailing of miRNA and small RNA is detected using a promer according to the present invention.

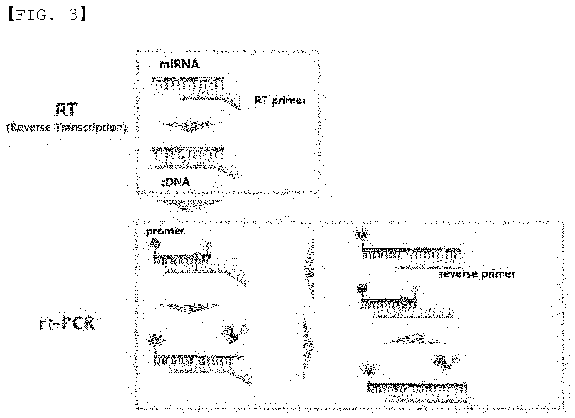

FIG. 3 is a schematic view showing a method in which miRNA is detected using a promer according to the present invention after synthesizing cDNA using an RT primer without poly-(A) tailing of the miRNA.
FIG. 4 is a graph showing the results of observing the expression of miRNA-21 gene using a promer according to the present invention.
FIG. 5 is a graph showing the results of observing the expression of miRNA-155 gene using a promer according to the present invention.
FIG. 6 shows the results of observing the expression of miRNA-21 cDNA using a promer according to the present invention after diluting the miRNA-21 cDNA to various concentrations.
FIG. 7 shows the results of observing the expression of miRNA-155 cDNA using a promer according to the present invention after diluting the miRNA-21 cDNA to various concentrations.
FIG. 8 is a graph showing the results of observing the expression of let-7a miRNA gene using a promer according to the present invention.
FIG. 9 is a graph showing the results of observing the expression of let-7d miRNA gene using a promer according to the present invention.
FIG. 10 shows the results of observing the expression of let-7a miRNA cDNA using a promer according to the present invention after diluting the let-7a miRNA cDNA to various concentrations in the presence of 1 pM let-7d.

### [Best Mode]

Hereinafter, the present invention will be described in detail with reference to examples. It will be obvious to those skilled in the art that these examples are merely intended to explain the present invention in more detail and the scope of the present invention according to the gist of the present invention is not limited by these examples.

### Example 1: Analysis of Human miRNA using Promer according to the Present Invention

In order to measure the expression of miRNA-21 gene (5' - uag cuu auc aga cug aug uug a) and miRNA-155 gene (5' - uua aug cua auc gug aua ggg gu), promers according to the present invention, primers and RT primers were synthesized by Integrated DNA Technologies (IDT, USA) (see Table 1). Here, the promers according to the present invention had FAM (fluorescein succinimidyl ester) attached to the 5' end and 3IABkFG attached to the 3' end, and ribonucleotides (RNA) were indicated by the subscript "r" in front of the sequences in order to distinguish them from deoxyribonucleotides (DNA).

**[Table 1]**

| | | |
|---|---|---|
| miRNA-21 promer | 5'-CCCTGTAGCTTATCAGACTGATrGTT | SEQ ID NO: 1 |
| miRNA-21 R primer | 5'-GCCGCTGAGGTAGTAGGT | SEQ ID NO: 2 |
| miRNA-21 RT primer | 5'-GCCGCTGAGGTAGTAGGTTGTGT(TTT)nTCA | SEQ ID NO: 3 |
| miRNA-155 promer | 5'-GGCTGTTAATGCTAATCGTGATCrGGG | SEQ ID NO: 4 |
| miRNA-155 R primer | 5'-GCCGCTGAGGTAGTAGGT | SEQ ID NO: 5 |
| miRNA-155 RT primer | 5'-GCCGCTGAGGTAGTAGGTTGTGT(TTT)nACC | SEQ ID NO: 6 |

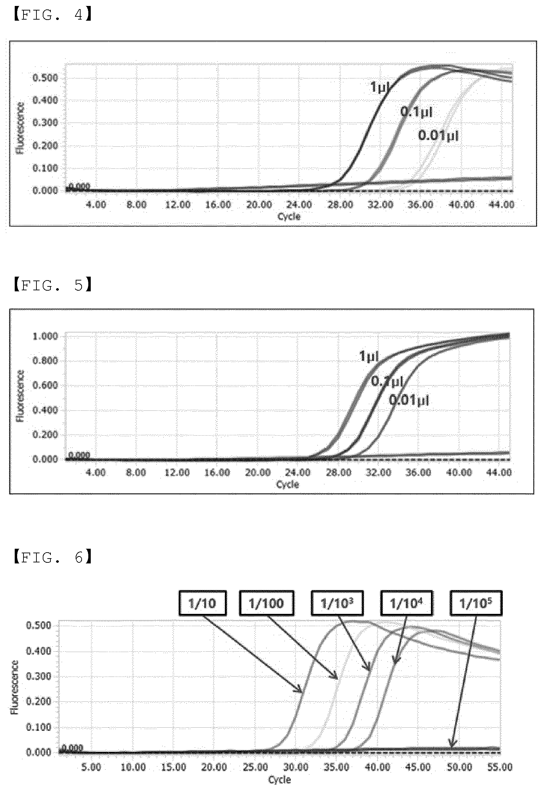

Using an exosomal miRNA purification kit (Genolution Co., Ltd.), 11.9 ng/µl miRNA was extracted from 200 µl of normal blood serum. Each of 1 µl, 0.1 µl and 0.01 µl of the extracted miRNA was allowed to react at 45°C in the presence of 1 µl of 10 µM each RT primer (shown in Table 1 above) in a poly- (A) tailing kit (Ambion) and a Nxtscript RT kit (total 20 µl, Roche), thereby synthesizing cDNAs. In the presence of 0.5 µl of each of 10 µM promer and 10 µM primer as shown in Table 1 above, 1 µl of each of the synthesized cDNAs, 10M units of heat-resistant RNase H, and 4 µl of AptaTaq DNA Master (manufactured by Roche) were added and the total volume was adjusted to 20 µl with triple distilled water, after which a polymerase chain reaction (PCR) was performed. At this time, the PCR reaction was performed under the following conditions: 5 min at 95°C, and then 45 cycles, each consisting of 60 sec at 63 to 64°C and 10 sec at 95°C. The results are shown in FIGS. 4 and 5.

As a result, it was confirmed that it was possible to perform stable analysis using only a very small amount (5.95 pg) of total miRNA, which corresponds to 1/20 of cDNA synthesized using only 119 pg of total miRNA.

### Example 2: Analysis of Human miRNA using Promer according to the Present Invention

cDNA synthesized from 1 µl of 11.9 ng/µl miRNA in Example 1 was diluted 1/10-fold, 1/100-fold, 1/1000-fold, 1/10000-fold, and 1/100000-fold.

In the presence of 0.5 µl of each of 10 µM promer and 10 µM primer as shown in Table 1 above, 1 µl of each of the diluted cDNAs, 10M units of heat-resistant RNase H, and 4 µl of AptaTaq DNA Master (manufactured by Roche) were added and the total volume was adjusted to 20 µl with triple distilled water, after which a polymerase chain reaction (PCR) was performed. At this time, the PCR reaction was performed under the following conditions: 5 min at 95°C, and then 55 cycles, each consisting of 60 sec at 63 to 64°C and 10 sec at 95°C. The results are shown in 6 and 7.

As a result, the amplification curve was plotted for the cDNA diluted up to 1/1000, and the amplification curve did not appear in the further diluted cDNA. This indicates that there was no nonspecific amplification in a very small amount of the target, suggesting that it would be possible to analyze less than 10 copies of a particular miRNA using only a very small amount of miRNA.

### Example 3: Real-Time Analysis of let-7 miRNA using Promer according to the Present Invention

let-7 miRNAs are known to have the largest number of isoforms among miRNAs. Distinguishing these isoforms of let-7 is known to be a very difficult analysis, and it is generally known that the distinction of a specificity of less than 1% is particularly difficult. In this Example, in order to measure the expression of let-7a gene (5' - uga ggu agu agg uug uau agu u) and let-7d gene (5' - aga ggu agu agg uug cau agu u) which are the most difficult among these isoforms, promers according to the present invention, primers and RT-primers were prepared by Integrated DNA Technologies (IDT, USA) as shown in Table 2 below (see Table 2). In addition, for accurate quantification, the miRNAs of let-7 were prepared by IDT. Here, the promers had FAM (fluorescein succinimidyl ester) attached to the 5' end and 3IABkFG attached to the 3' end, and ribonucleotides (RNA) were indicated by the subscript "r" in front of the sequences in order to distinguish them from deoxyribonucleotides (DNA).

**[Table 2]**

| | | |
|---|---|---|
| let-7a promer | 5'-GCTGCTTGAGGTAGTAGGTTGrUAT | SEQ ID NO: 7 |
| let-7a R primer | 5'-GCCGCTGAGGTAGTAGGT | SEQ ID NO: 8 |
| let-7a RT primer | 5'-GCTAACGTCTGTACTTCGTCA(TTT)nAACT | SEQ ID NO: 9 |
| let-7d promer | 5'-GCTGCTAGAGGTAGTAGGTTGrCAT | SEQ ID NO: 10 |
| let-7d R primer | 5'-GCCGCTGAGGTAGTAGGT | SEQ ID NO: 11 |
| let-7d RT primer | 5'-GCTAACGTCTGTACTTCGTCA(TTT)nAACT | SEQ ID NO: 12 |

Using 1 µl of 20 pM each synthesized miRNA, a poly- (A) tailing kit (Ambion) and a Nxtscript RT kit (Roche) (total 20 µl), a reaction was performed at 45°C for 30 minutes in the presence of 1 µl of 10 µM each RT primer shown in Table 2 above, thereby synthesizing cDNAs.

The synthesized cDNAs were diluted from a concentration of 100 fM to a concentration of 1 aM.

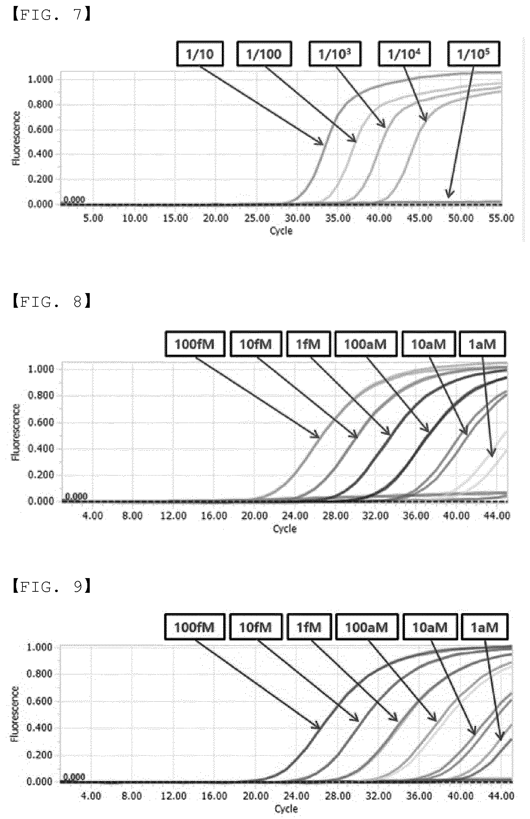

In the presence of 0.5 µl of each of 10 µM promer and 10 10 µM primer as shown in Table 2 above, 2 µl of each of the synthesized and diluted cDNAs, 10M units of heat-resistant RNase H, and 4 µl of AptaTaq DNA Master (manufactured by Roche) were added and the total volume was adjusted to 20 µl with triple distilled water, after which a polymerase chain reaction (PCR) was performed. At this time, the PCR reaction was performed under the following conditions: 5 min at 95°C, and then 45 cycles, each consisting of 60 sec at 63 to 64°C and 10 sec at 95°C. The results are shown in FIGS. 8 and 9.

As a result, it was confirmed that it was possible to analyze each miRNA using only 2 µl (about 1 copy) of cDNA diluted to a concentration of 1 aM.

### Example 4: Real-Time Detection of let-7 miRNA Specificity using Promer according to the Present Invention

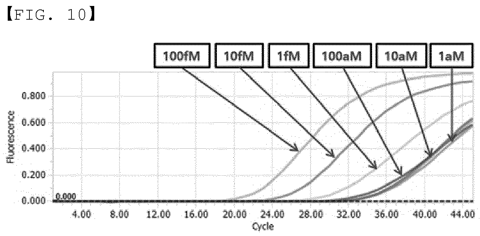

In the presence of 0.5 µl of each of 10 µM promer and 10 µM primer as shown in Table 2 above, 2 µl of each let-7a cDNA diluted at 1/10 (from a concentration of 100 fM to 1 aM) was added to 2 µl of 1 pM each let-7a cDNA constructed in Example 3 above, and 10M units of RNase H and 4 µl of AptaTaq DNA Master (manufactured by Roche) were added. The total volume was adjusted to 20 µl with triple distilled water, after which a polymerase chain reaction (PCR) was performed. At this time, the PCR reaction was performed under the following conditions: 5 min at 95°C, and then 45 cycles, each consisting of 60 sec at 63 to 64°C and 10 sec at 95°C. The results are shown in FIG. 10.

As a result, it was confirmed that stable analysis was possible at a concentration ranging from 100 fM to 1 fM in the test group in which 2 µl of each let-7a cDNA diluted at 1/10 (from a concentration of 100 fM to 1 aM) was added to 2 µl of 1 pM each let-7a cDNA, but the graph fell down at 100 aM. This suggests that the isoforms of miRNA could be analyzed while the specificity was maintained up to 0.1%.

## Claims

1. A method for detecting small RNA, either comprising the steps of:
a) obtaining an RNA including the small RNA to be detected from a biological sample; b) synthesizing a cDNA from the RNA obtained in step a) by use of a promer having the structure X-Y-Z, which comprises a nucleotide sequence capable of binding complementarily to a portion of the nucleotide sequence of the small RNA to be detected; c) amplifying the cDNA synthesized in step b); and d) measuring the amount of a fragment of the promer cleaved by an enzyme capable of cleaving region Y of the promer of step b);
or comprising the steps of: a-1) an RNA including the small RNA to be detected from a biological sample; b-1) synthesizing a cDNA from the RNA obtained in step a-1); c-1) adding, to the cDNA of step b-1), a promer which comprises a nucleotide sequence capable of binding complementarily to a portion of the nucleotide sequence of the cDNA and is capable of being used as (i) a forward primer and a probe, or (ii) a reverse primer or a probe, or (iii) a forward primer, a reverse primer and a probe, and amplifying the cDNA; d-1) measuring the amount of a fragment of the promer cleaved by an enzyme capable of cleaving region Y of the promer of step c-1),
wherein the promer has the structure X-Y-Z; one or more detectable markers are attached at both ends or inside of the promer and form a complex by binding to a specific region of a target nucleic acid or target protein to be detected in real time; and when region Y is cleaved by a specific enzyme, then region X acts as a primer while maintaining the complex with the target nucleic acid or target protein, and Y and Z are separated from the specific region of the target nucleic acid or target protein;
each of X, Y and Z is a DNA or RNA comprising nucleotides; and
when any one of X, Y and Z is DNA, then one or more of the other two are RNA.

2. The method of claim 1, wherein
X is a DNA or RNA comprising 1 to 60 nucleotides;
Y is a DNA or RNA comprising 1 to 10 nucleotides; and
Z is a DNA or RNA comprising 0 to 10 nucleotides;
when Z is 0, then Y is a DNA or RNA comprising 3 to 10 nucleotides; and
when Z is 1, then Y is a DNA or RNA comprising 2 to 10 nucleotides.

3. The method of claim 1, wherein the detectable marker is either a fluorophore that binds covalently or non-covalently to the promer, or a fluorescent pair consisting of the fluorophore and a quencher.

4. The method of claim 1, wherein X, Y and Z of the promer are synthesized to be completely or partially methylated in order to prevent nonspecific cleavage.

5. The method of claim 1, wherein X of the promer is a DNA or RNA comprising 10 to 30 nucleotides, Y is a DNA or RNA comprising 1 to 10 nucleotides, and Z is a DNA or RNA comprising 2 to 10 nucleotides.

6. The method of claim 1, wherein when region Y is DNA, then the enzyme is DNA nuclease (DNase), specifically, DNase I, DNase II, S1 nuclease, nuclease P1, AP endonuclease, or UvrABC nuclease, and when region X is RNA, then the enzyme is RNA ribonuclease (RNase), specifically, RNase II, RNase III, RNase IV, RNase H, or RNase T2.

7. The method of claim 1, wherein, in step c) or step c-1), region Y of the promer bound to the cDNA synthesized in step b) or step b-1) is cleaved by an enzyme that cleaves region Y of the promer, and thus regions Y and Z are separated, and region X acts as a primer and is amplified.

8. The method of claim 1, wherein, in step c-1), when (i) the promer is used as the forward primer and the probe, it further comprises a reverse primer comprising a nucleotide sequence capable of binding complementarily to a portion of the nucleotide sequence of the cDNA, and when (ii) the promer is used as the reverse primer and the probe, it further comprises a forward primer comprising a nucleotide sequence capable of binding complementarily to a portion of the nucleotide sequence of the cDNA.

9. A method for detecting a small RNA-associated protein molecule, comprising the steps of: a) producing an antibody having attached thereto a nucleic acid having a nucleotide sequence complementary to a promer having the structure X-Y-Z; b) combining the antibody produced in step a) with a small RNA-associated protein obtained from a biological sample, thereby forming a protein-antibody complex; c) hybridizing the promer having the structure X-Y-Z to the complex, thereby forming a protein-antibody-promer complex; and d) treating the protein-antibody-promer complex with a cleavage reagent, thereby separating a fragment of the promer from the antibody, and then measuring the amount of the separated promer, thereby detecting the small RNA-associated protein molecule,
wherein the promer has the structure X-Y-Z; one or more detectable markers are attached at both ends or inside of the promer and form a complex by binding to a specific region of a target nucleic acid or target protein to be detected in real time; and when region Y is cleaved by a specific enzyme, then region X acts as a primer while maintaining the complex with the target nucleic acid or target protein, and Y and Z are separated from the specific region of the target nucleic acid or target protein;
each of X, Y and Z is a DNA or RNA comprising nucleotides; and
when any one of X, Y and Z is DNA, then one or more of the other two are RNA.

10. The method of claim 9, wherein, in step d), region Y of the promer bound to the antibody produced in step a) is cleaved by an enzyme that cleaves region Y of the promer, and thus regions Y and Z are separated, and region X acts as a primer and is amplified.
